Europäisches Patentamt

European Patent Office

Office européen des brevets

(11) Veröffentlichungsnummer: **0 491 979 A1**

## EUROPÄISCHE PATENTANMELDUNG

(21) Anmeldenummer: **90125337.7**

(22) Anmeldetag: **22.12.90**

(51) Int. Cl.5: **A61N 1/05**

(43) Veröffentlichungstag der Anmeldung:
**01.07.92 Patentblatt 92/27**

(84) Benannte Vertragsstaaten:
**AT CH DE ES FR GB IT LI NL SE**

(71) Anmelder: **Osypka, Peter, Dr. Ing.
Basler Strasse 109
W-7889 Grenzach-Wyhlen(DE)**

(72) Erfinder: **Osypka, Peter, Dr. Ing.
Basler Strasse 109
W-7889 Grenzach-Wyhlen(DE)**

(74) Vertreter: **Patentanwälte Dipl.-Ing. Hans
Schmitt Dipl.-Ing. Wolfgang Maucher
Dreikönigstrasse 13
W-7800 Freiburg i.Br.(DE)**

(54) **Herzschrittmacher-Katheter mit zwei Polen.**

(57) Ein Herzschrittmacher-Katheter (1) mit wenigstens zwei in axialer Richtung beabstandeten Polen und mit innerhalb von Isolierungen zu diesen Polen führenden Wendeln (3 und 4) oder dergleichen Zuleitungen hat zumindest einen ersten Pol (2), der durch einen Metallring oder eine Pol-Hülse (5) gebildet und mit der entsprechenden Zuleitung elektrisch leitend verbunden ist. Diese Verbindung ist durch wenigstens ein elektrisch leitendes Metallband (6) hergestellt, welches einerseits mit der zu der Pol-Hülse (5) gehörenden Zuleitung oder Wendel (6) und andererseits mit der Pol-Hülse (5) verbunden ist, so daß eine Löt- oder Schweißverbindung für eine sichere Kontaktgabe hoher Festigkeit möglich ist und durch die Flexibilität des Metallbandes auch dynamische Belastungen im Pol-Bereich ohne Auswirkung auf diese elektrische Verbindung bleiben.

EP 0 491 979 A1

Die Erfindung betrifft einen Herzschrittmacher-Katheter mit zumindest zwei voneinander in axialer Richtung beabstandeten Reizelektroden oder Polen sowie mit zumindest zwei innerhalb einer Isolierung zu den Polen geführten, zu einer Wendel gewickelten und gegeneinander isolierten Zuleitungen, deren Windungen jeweils parallel und koaxial zueinander angeordnet sind, wobei ein erster Pol durch einen leitenden Metallring oder eine Pol-Hülse gebildet ist, der mit einer der Zuleitungen elektrisch leitend verbunden ist und gegenüber den Zuleitungen zu dem zweiten Pol isoliert ist.

Ein derartiger Herzschrittmacher-Katheter ist aus der DE-PS 30 43 189 C 2 bekannt. Bei demjenigen Ausführungsbeispiel gemäß dieser Druckschrift, bei welchem eine Hülse den proximalen Pol bildet, werden die Enden der Zuleitungen zu diesem Pol durch die Isolierung nach außen geführt, um mit der Hülse verbunden werden zu können.

Bei einer Herzschrittmacher-Elektrode gemäß der DE-A-37 18 324 soll dies dadurch vermieden werden, daß Leiter unterschiedlicher Querschnitte benutzt werden, so daß nach Entfernen der Ummantelung im Bereich des Poles die Hülse auf die Leiter größeren Durchmessers aufgeschoben und dort festgeklemmt werden kann. Dies bedeutet jedoch, daß eine Wendel aus Leitern gebildet werden muß, die unterschiedliche Querschnitte und damit auch unterschiedliche Außendurchmesser haben. Ferner ergeben sich durch diese unterschiedlichen Durchmesser innerhalb der Ummantelung durch das sich bewegende Herz Stabilitätsprobleme. Ferner ist der Druckkontakt zwischen Hülse und blanken Leitern keine zuverlässige elektrische Verbindung. Auch läßt sich eine Kurzschlußgefahr nicht ausschließen, weil unter Umständen durch die Bewegungen auch einmal Leiter geringeren Durchmessers mit der Pol-Hülse in Berührung kommen könnten.

Aus der EP-A-0 292 596 ist ein Herzschrittmacher-Katheter bekannt, bei welchem ein gewünschter Poldraht selektiv mit Hilfe eines speziellen Werkzeuges durch die isolierende Ummantelung nach außen gezogen und zwischen zwei elektrisch leitenden Hülsen eingepreßt oder angeschweißt wird. Eine solche Quetschverbindung eines elektrischen Leiters zwischen zwei Metallhülsen ist aufgrund der Abschergefahr sehr riskant. Vor allem ist dabei eine Kontrolle der Zuverlässigkeit insbesondere an den Kanten der Innenhülse, an denen der Leiter umgebogen werden muß, praktisch ausgeschlossen. Dabei ist wiederum zu berücksichtigen, daß die Anordnung ständigen Bewegungen durch den Herzschlag und die Körperbewegungen des Benutzers ausgesetzt ist.

Es besteht deshalb die Aufgabe, einen implantierbaren Herzschrittmacher-Katheter der eingangs erwähnten Art so auszubilden, daß eine sichere elektrisch leitende Verbindung zwischen dem proximalen Pol und seiner Zuleitung erfolgt und trotz der ständigen Bewegungen aufrechterhalten bleibt.

Die Lösung dieser Aufgabe besteht darin, daß wenigstens ein elektrisch leitendes Band einerseits in elektrischem Kontakt mit Windungen der zu der Pol-Hülse gehörenden Wendel vorgesehen und andererseits mit dieser Pol-Hülse verbunden ist.

Ein solches Metallband ergibt einerseits aufgrund seiner relativ großen Breite eine sichere und gute Kontaktfläche, führt andererseits aber aufgrund seiner relativ geringen Dicke nicht zu der Gefahr eines Abquetschens oder Abscherens, sondern läßt sich gut im Inneren der Pol-Hülse einpassen und anbringen; hinzu kommt, daß ein Band aufgrund der relativ geringen Dicke im Verhältnis zur Breite eine gute Flexibilität hat, die eine spannungsfreie Verbindung der zu kontaktierenden Stellen mit einer entsprechenden Eigenbeweglichkeit kombiniert, so daß es auch unter den Bewegungen des Herzens und des Trägers nicht zu Zugspannungen innerhalb dieses Bandes und an seinen Kontaktstellen kommen kann.

Besonders zweckmäßig ist es für eine stabile Ausgestaltung des proximalen Poles, wenn innerhalb der diesen Pol bildenden Pol-Hülse eine Stützhülse angeordnet ist, mit der das innere Ende des Metallbandes elektrisch leitend verbunden ist, und wenn das Metallband zwischen einem etwa in axialer Richtung an dieser Haltehülse verlaufenden Abschnitt und einem zweiten, an der Innenseite der Pol-Hülse verlaufenden Abschnitt einen etwa S-förmigen Übergang aufweist. Dabei ist natürlich die Stützhülse mit der entsprechenden Zuleitung zu diesem proximalen Pol ihrerseits elektrisch leitend verbunden. Der S-förmige Übergang schafft genügend Flexibilität innerhalb der gesamten Verbindung, um Zugbelastungen an dem Metallband selbst beziehungsweise seinen Kontaktstellen auszuschließen. Außerdem lassen sich so die unterschiedlichen radialen Ausdehnungen der beiden ineinander angeordneten Hülsen problemlos überbrücken.

Die innere Haltehülse kann etwa gleich lang oder wenig kürzer oder länger als die Pol-Hülse sein und als innere Armierung des Polbereiches insbesondere beim Aufpressen der Pol-Hülse dienen. Somit erhält diese Haltehülse eine Doppelfunktion, weil sie einerseits den Pol vor allem auch beim Aufpressen der Pol-Hülse sowie auch später stabilisiert und andererseits als guter Kontakt für das Metallband dienen kann.

Eine Ausführungsform des erfindungsgemäßen Herzschrittmacher-Katheters kann vorsehen, daß für jeden der Pole eine eigene, vorzugsweise wendelförmige Zuleitung vorgesehen ist und beide Wendeln koaxial ineinander verlaufen, wobei eine Isolierung zwischen ihnen angeordnet ist, daß die auf größerem Durchmesser verlaufende Wendel zu dem proximalen, als Pol-Hülse gestalteten Pol gehört und in ihrem Endbereich innerhalb des Poles die Haltehülse zwischen sich und der Isolierung zu der inneren Wendel aufnimmt und daß das Metallband zwischen dieser äußeren Wendel und der Haltehülse an der Haltehülse und insbesondere mehreren Windungen dieser Wendel anliegt und zwischen zwei Windungen und durch die äußere Isolierung der äußeren Wendel an die Innenseite der Pol-Hülse geführt ist. Sind also koaxial ineinander verlaufende wendelförmige Zuleitungen vorgesehen, läßt sich die außenlaufende Wendel sehr einfach mit der Haltehülse verbinden, indem diese zwischen die Isolierung der inneren Wendel und die äußere Wendel eingeschoben oder die äußere Wendel auf diese Haltehülse mit aufgewickelt wird. Dabei kann dann das Metallband gleich von der Wendel mitumschlossen und gegen die Haltehülse gepreßt werden. Zwischen zwei Windungen dieser Wendel kann dann das Metallband wiederum etwa S- oder Z-förmig nach außen geführt sein, um mit der Innenseite der Pol-Hülse Kontakt zu erhalten.

Eine andere Ausgestaltung des erfindungsgemäßen implantierbaren Herzschrittmacher-Katheters kann vorsehen, daß die Windungen der beiden Zuleitungen zu einer gemeinsamen Mehrfachwendel gewickelt sind, deren Windungen alle denselben Außendurchmesser aufweisen und gegeneinander isoliert sind, daß im Bereich des proximalen Poles die Haltehülse innerhalb der Mehrfachwendel angeordnet ist, daß die zu dem proximalen Pol führende Zuleitung im Bereich dieser Haltehülse abisoliert und mit dieser elektrisch leitend verbunden ist und daß das Metallband mit dieser Haltehülse außenseitig verbunden und zwischen isolierten Windungen radial nach außen an die Innenseite der äußeren Pol-Hülse geführt ist.

In beiden Fällen ist also die Haltehülse jeweils innerhalb von Windungen der Zuleitung angeordnet und stützt diese im Polbereich ab, wobei die beim Aufpressen der Pol-Hülse über die Windungen auf diese Stütz- oder Haltehülse wirkenden Kräfte zur Verbesserung des gegenseitigen Kontaktes und vor allem des Kontaktes mit dem dazwischenliegenden Metallband beitragen.

Im Bereich der inneren Haltehülse kann zwischen dieser und den Windungen ein etwa über die Länge der Haltehülse reichender Isolier-Überzug vorgesehen sein, der Im Bereich der Verbindung mit dem Ende der Zuleitung zu der Haltehülse durchbrochen ist, und das Metallband kann zwischen dieser Isolierung und der Außenseite der Haltehülse angeorndet sein und durch die Isolierung hindurch nach außen verlaufen. Somit wird durch die Isolierung der Haltehülse gleichzeitig auch das Metallband soweit isoliert, wie es im Kontakt mit der Haltehülse ist.

Eine wiederum abgewandelte Ausgestaltung des implantierbaren Herzschrittmacher-Katheters gemäß der Erfindung kann vorsehen, daß die innere Haltehülse an der Außenseite der gemeinsamen Isolierung der als Mehrfachwendel gewickelten Windungen der verschiedenen Zuleitungen angeordnet ist, daß die zu dem proximalen Pol führenden Windungen durch die Isolierung hindurchgeführt und mit der Außenseite dieser Stütz- oder Haltehülse verbunden sind, mit der gleichzeitig das Metallband verbunden ist, und daß das Metallband durch eine äußere Isolierung der Haltehülse zu der Innenseite der Pol-Hülse geführt ist.

Alle Ausführungsbeispiele erlauben eine besonders vorteilhafte Ausgestaltung dahingehend, daß das Metallband mit der Haltehülse und/oder der Innenseite der Pol-Hülse verschweißt ist. Dies läßt sich besonders gut bewerkstelligen, wenn das Metallband aus demselben Metall wie die Hülsen besteht. Andernfalls ist aber auch ein Verlöten möglich, um eine feste und vor allem gut leitfähige Verbindung herzustellen.

Die Löt- oder Schweißstelle zwischen der Pol-Hülse und dem Metallband kann dabei nahe dem stirnseitigen Rand der Pol-Hülse angeordnet sein, wo das Metallband endet. Diese Stelle ist trotz der Anordnung des Metallbandes an der Innenseite der Pol-Hülse gut zugänglich, um hier eine Schweißung vornehmen zu können.

Ferner kann der Kontakt und gleichzeitig die Festigkeit der jeweiligen Verbindung bei allen Ausführungsbeispielen dadurch gesteigert sein, daß das Ende der zu dem proximalen Pol führenden Zuleitung oder Zuleitungen mit der Stütz- oder Haltehülse verschweißt ist.

Die Erfindung läßt sich auch bei einem Herzschrittmacher-Katheter mit mehr als zwei Polen dadurch realisieren, daß eine weitere, wenigstens aus einem Draht gewickelte Zuleitung vorgesehen ist, die zu einem dem proximalen Pol benachbarten weiteren Pol führt und über ein weiteres Metallband und vorzugsweise eine innere Stützhülse mit einer äußeren Pol-Hülse verbunden ist. Die erfindungsgemäße Kontakt-Verbindung zwischen Zuleitung und Pol-Hülse kann also an einem entsprechend mehrpoligen Herzschrittmacher-Katheter mehrfach wiederholt sein.

Insgesamt ergibt sich eine Konstruktion des proximalen Poles, die eine hohe Sicherheit der elektrischen Verbindung zwischen Zuleitung und Pol-Hülse bei gleichzeitig guter Stabilität dieser Verbindung aufgrund des dünnen und flachen, aber beliebig breiten Metallbandes gewährleistet.

Nachstehend sind mehrere Ausführungsbeispiele der Erfindung anhand der Zeichnung noch näher beschrieben, wobei jeweils nur der Teil des Herzschrittmacher-Katheters dargestellt ist, der den proximalen Pol aufweist.

Im einzelnen zeigt:

Fig. 1

einen Ausschnitt aus einer erfindungsgemäßen Herzschrittmacher-Leitung im Bereich eines proximalen Poles, wobei die Zuleitungen zu den Polen als koaxial ineinander verlaufende und gegeneinander isolierte Wendeln ausgebildet sind und im Bereich des Poles auf der Isolierung der inneren Wendel eine Stütz- oder Haltehülse befestigt ist, an deren Außenseite ein Metallband elektrischen Kontakt hat und auf welcher Außenseite über den Befestigungsbereich des Metallbandes hinweg die Windungen der koaxial äußeren Wendel als Zuleitung zu diesem proximalen Pol verläuft, die außerdem an dieser Haltehülse angeschweißt ist und ihrerseits eine äußere Isolierung hat, durch welche das Metallband nach außen an die Innenseite einer Pol-Hülse geführt ist,

Fig. 2 bis Fig. 4

den allmählichen Aufbau des proximalen Poles gemäß Fig. 1 von der Anbringung der Haltehülse an der Isolierung der inneren Wendel mit Metallband (Fig.2) über die Anbringung der äußeren Wendel auf der Außenseite der Haltehülse (Fig. 3) bis zur Anbringung der äußeren Isolierung für diese äußere Wendel (Fig. 4), wonach die Pol-Hülse noch aufzuschieben und/oder aufzupressen ist,

Fig. 5

eine abgewandelte Ausführungsform eines erfindungsgemäßen Herzschrittmacher-Katheters, bei welchem die Wendeln der unterschiedlichen Zuleitungen zu den Polen als Mehrfachwendel mit gleichem Durchmesser nebeneinander gewickelt sind und im Bereich des Poles eine gegenüber den Windungen der Wendeln isolierte Haltehülse vorgesehen ist, an deren Außenseite das Metallband befestigt, zwischen den Windungen nach außen durch die äußere Isolierung der Wendeln an die Innenseite der Pol-Hülse geführt ist, sowie

Fig. 6

eine wiederum abgewandelte Ausführungsform eines proximalen Poles eines erfindungsgemäßen Herzschrittmacher-Katheters, wobei parallel zueinander und gegeneinander isolierte Windungen zweier Wendeln mit gleichem Außendurchmesser und gemeinsamer äußerer Isolierung vorgesehen sind, an deren Außenseite die Haltehülse angeordnet und mit einer weiteren Isolierung von der Innenseite der Pol-Hülse getrennt ist.

Ein in allen Figuren jeweils nur bezüglich eines Ausschnittes dargestellter Herzschrittmacher-Katheter 1 hat zumindest zwei voneinander in axialer Richtung beabstandete Reizelektroden oder Pole, von denen jeweils der proximale Pol 2 dargestellt ist. Zu jedem Pol führt innerhalb einer Isolierung eine zu einer Wendel gewickelte Zuleitung, wobei in allen Ausführungsbeispielen die zu dem Pol 2 führende Wendel mit 3 und die zu einem weiteren, zum Beispiel distalen Pol führende Wendel mit 4 bezeichnet ist. Der erste oder proximale Pol 2 ist in allen Ausführungsbeispielen durch einen leitenden Metallring oder eine Pol-Hülse 5 gebildet, wobei allerdings zu dem Pol 2 außerdem die elektrische Verbindung zu seiner Wendel 3 gehört. Der Pol 2 ist dabei außerdem gegenüber der Zuleitung 4 zu dem nicht näher dargestellten zweiten Pol isoliert.

In allen Ausführungsbeispielen ist ein elektrisch leitendes Metallband 6 einerseits in elektrischen Kontakt mit Windungen der zu der Pol-Hülse 5 gehörenden Wendel 3 vorgesehen und andererseits mit der Innenseite dieser Pol-Hülse 5 verbunden.

Für den elektrischen Kontakt mit der Wendel 3 sind dabei unterschiedliche Möglichkeiten vorgesehen. In allen Beispielen ist dazu innerhalb der Pol-Hülse 5 eine Stütz- oder Haltehülse 7 angeordnet, mit der das innere Ende des Metallbandes 6 elektrisch leitend verbunden ist. Das Metallband 6 hat zwischen einem etwa in axialer Richtung an dieser Haltehülse 7 verlaufenden Abschnitt 6a und einem zweiten, an der Innenseite der Pol-Hülse 5 verlaufenden Abschnitt 6b einen etwa S- oder Z-förmigen Übergang, wodurch die unterschiedlichen Durchmesser, auf denen sich die Abschnitte 6a und 6b befinden, überbrückt werden. Auf diese Weise kann also zwischen der innenliegenden Haltehülse 7 und der außenliegenden Pol-Hülse 5 eine Verbindung hergestellt werden. Da gleichzeitig die Haltehülse 7 elektrischen Kontakt mit der Wendel 3 hat, ist so die Verbindung zu der Pol-Hülse 5 sichergestellt, wobei dieser elektrische Kontakt unabhängig von Reibschluß oder Klemmkräften zwischen einer Hülse und den Drahtwindungen der Wendel 3 ist.

Man erkennt auch in allen Ausführungsbeispielen, daß die innere Haltehülse 7 etwa gleich lang wie die Pol-Hülse 5 ist und koaxial zu dieser angeordnet ist, so daß sie auch eine Armierung des Pol-Bereiches insbesondere beim Aufpressen der Pol-Hülse 5 bildet. Sie könnte eventuell auch etwas kürzer oder etwa länger als die Pol-Hülse 5 sein.

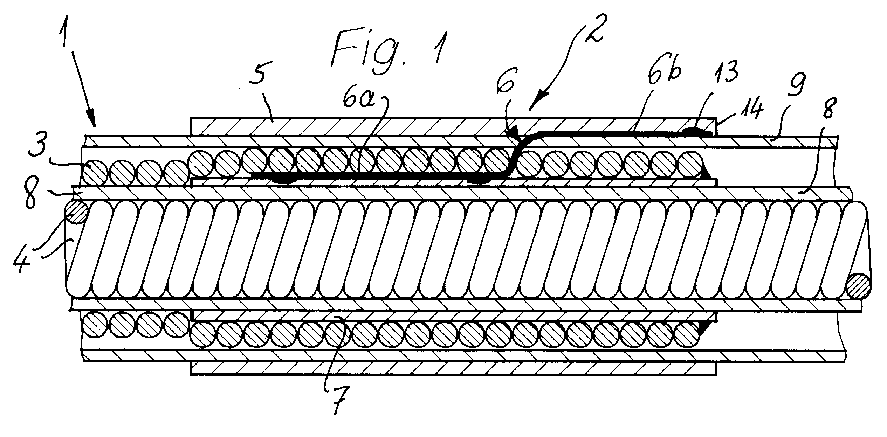

Bei dem Ausführungsbeispiel gemäß den Figuren 1 bis 4 ist für jeden der Pole eine eigene wendelförmige Zuleitung 3 und 4 vorgesehen und beide Wendeln 3 und 4 verlaufen koaxial ineinander, d.h. auf unterschiedlichen Durchmessern. Zwischen beiden Wendeln 3 und 4 ist eine schlauchförmige Isolierung 8 angeordnet, so daß die beiden Wendeln 3 und 4 in vorteilhafter Weise nicht selbst isoliert sein müssen, sondern aus blankem Draht bestehen können. Die auf größerem Durchmesser verlaufende Wendel 3 gehört zu dem proximalen, als Pol-Hülse 5 gestalteten Pol und nimmt in ihrem Endbereich innerhalb des Poles 2 die Haltehülse 7 zwischen sich und der Isolierung 8 auf. Das Metallband 6 liegt mit seinem Abschnitt 6a zwischen dieser äußeren Wendel 3 und der Haltehülse 7 an deren Außenseite und mehreren Windungen der Wendel 3 an, weil es sich zwischen der Haltehülse 7 und dieser Wendel 3 befindet. Zwischen zwei Windungen ist das Metallband 6 mit seinem S-förmigen Übergang durch die äußere Isolierung 9 der äußeren Wendel 3 an die Innenseite der Pol-Hülse 5 geführt.

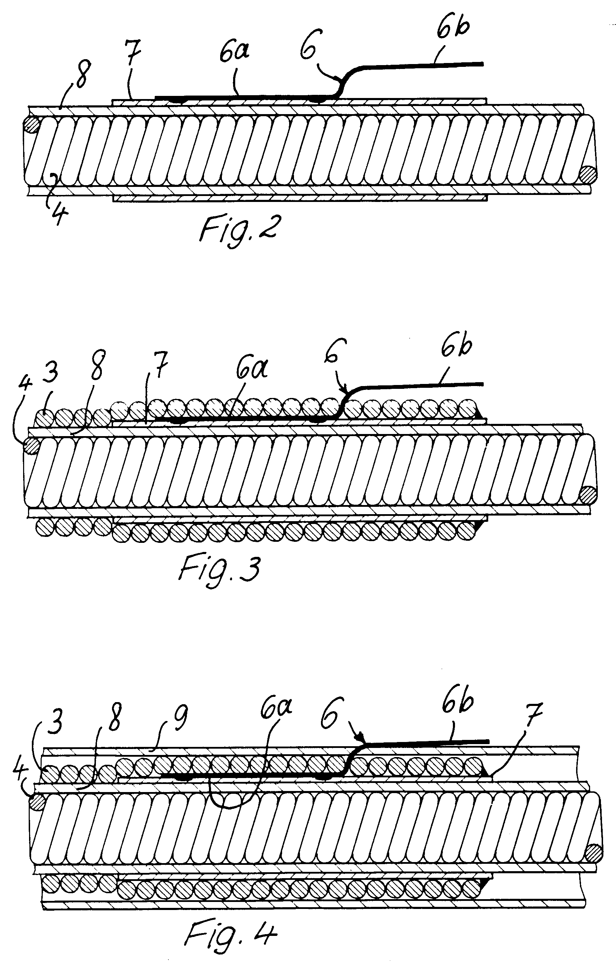

Dieser Aufbau wird ganz deutlich bei Betrachtung der Figuren 2 bis 4. In Fig. 2 erkennt man zunächst den Polbereich nur daran, daß auf der inneren Wendel 4 und deren Isolierung 8 die Haltehülse 7 und das mit dieser verbundene Metallband 6 angebracht ist.

Fig. 3 zeigt zusätzlich zu Fig. 2 die äußere Wendel 3 als Zuleitung zu dem noch nicht komplettierten Pol 2, die elektrisch leitenden Kontakt mit der Haltehülse 7 und dem Metallband 6 hat. Fig. 4 zeigt zusätzlich die äußere Isolierung 9 der Wendel 3, durch welche das Metallband 6 und sein Abschnitt 6b nach außen geführt sind. Wird anschließend die Pol-Hülse 5 angebracht, ergibt sich der Gesamtaufbau gemäß Fig. 1.

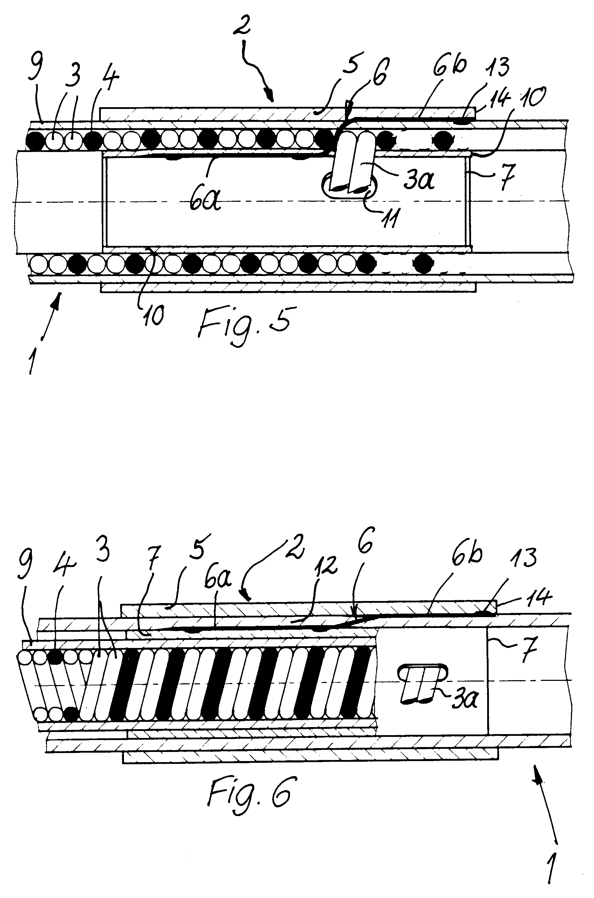

Beim Ausführungsbeispiel nach Fig. 5 sind die Windungen der beiden Zuleitungen zu einer gemeinsamen Mehrfachwendel gewickelt, deren Windungen alle denselben Außendurchmesser aufweisen und beispielsweise durch Benutzung von isoliertem Draht gegeneinander isoliert sind. Die weiß dargestellten Windungen 3 gehören dabei zu der Zuleitung zu dem proximalen Pol 2, während die schwarz dargestellten Windungen 4 zu dem nicht dargestellten distalen Pol weiterlaufen, was in Fig. 5 der Einfachheit halber nicht näher dargestellt ist.

Im Bereich des proximalen Poles 2 ist innerhalb dieser Mehrfachwendel die Haltehülse 7 angeordnet und die zu diesem proximalen Pol 2 führende Zuleitung 3 ist im Bereich der Haltehülse 7 abisoliert, sofern sie nicht von vorneherein aus blankem Draht besteht und nur die Zuleitung 4 isoliert ist. Die Zuleitung 3 ist mir ihren Endstücken 3a elektrisch leitend mit der Haltehülse 7 verbunden, zweckmäßigerweise verschweißt, und das Metallband 6 ist wiederum an der Haltehülse 7 außenseitig mit dieser in Kontakt und verläuft zwischen zumindest teilweise isolierten Windungen radial nach außen zu der Innenseite der äußeren Pol-Hülse 5.

Dabei erkennt man in Fig. 5 außerdem im Bereich der inneren Haltehülse 7 zwischen dieser und den Windungen der Zuleitungen 3 und 4, von denen einige nicht isoliert sind, einen etwa über die Länge der Haltehülse 7 reichenden Isolier-Überzug 10, der im Bereich der Verbindung mit den Enden 3a der Zuleitung einen Durchbruch 11 hat, um den elektrischen Kontakt zu der Zuleitung 3 zu ermöglichen. Das Metallband 6 befindet sich mit seinem Abschnitt 6a zwischen diesem Isolier-Überzug 10 und der Außenseite der Haltehülse 7 und verläuft somit auch durch den Isolier-Überzug 10 hindurch nach außen.

Auch beim Ausführungsbeispiel nach Fig. 6 sind die Windungen 3 und 4 der Zuleitungen zu den Polen koaxial zueinander nebeneinander als Mehrfachwendel gestaltet und gegeneinander isoliert. Die innere Haltehülse 7 ist dabei an der Außenseite der gemeinsamen äußeren Isolierung 9 - die auch beim Ausführungsbeispiel nach Fig. 5 vorgesehen ist - angeordnet. Demgemäß ist das Ende oder sind die Enden der zu dem proximalen Pol 2 führenden Windungen 3, die in Fig. 6 wiederum weiß dargestellt sind und aus blankem Draht bestehen können, durch die Isolierung 9 hindurch und außerdem durch einen Durchbruch 11 auf die Außenseite der Haltehülse 7 geführt und mit dieser verbunden, wobei mit der Außenseite der Haltehülse 7 auch das Metallband 6 verbunden ist, welches durch eine äußere Isolierung 12 der Haltehülse 7 zur Innenseite der Pol-Hülse 5 geführt ist.

In allen Ausführungsbeispielen ist für eine feste Verbindung und einen guten elektrischen Kontakt vorgesehen, daß das Metallband 6 mit der Haltehülse 7 und mit der Innenseite der Pol-Hülse 5 verschweißt oder bei der Verwendung unterschiedlicher Metalle verlötet ist. Dabei erkennt man, daß die Löt- oder Schweißstelle 13 zwischen der Pol-Hülse 5 und dem Metallband 6 nahe dem stirnseitigen Rand 14 der Pol-Hülse 5 angeordnet ist, wo das Metallband 6 endet, so daß diese Schweißung trotz der Anordnung an der Innenseite der Pol-Hülse 5 gut durchgeführt werden kann. Die Verschweißung mit der Außenseite der Haltehülse 7 kann praktisch schon unmittelbar nach dem Anbringen der Haltehülse 7 oder gar vor deren Montage durchgeführt werden.

Außerdem sind in allen Ausführungsbeispielen das oder die Enden der zu dem proximalen Pol 2 führenden Zuleitung oder Zuleitungen mit der Haltehülse 7 verschweißt, so daß der Stromfluß an keiner Stelle nur über Klemm- oder Reibkontakte erfolgen muß. Es entsteht also eine bestmögliche elektrische Verbindung, die gleichzeitig eine hohe Festigkeit hat, wobei das Metallband aufgrund seiner flachen, aber relativ breiten Querschnittsform eine genügende Flexibilität hat, um auch bei ständigen Bewegungen des Poles 2 durch den Herzschlag oder den Träger keinen gefährlichen Zugbelastungen ausgesetzt zu werden.

Es sei noch erwähnt, daß selbstverständlich eine weitere, wenigstens aus einem Draht gewickelte Zuleitung vorgesehen sein könnte, die zu einem dem proximalen Pol 2 benachbarten weiteren Pol führt, dem dann eventuell der distale Pol benachbart ist, wobei wiederum eine innere Stütz- oder Haltehülse 7 mit einer äußeren Pol-Hülse 5 durch ein weiteres Metallband 6 verbunden sein könnte, d.h. die anhand des Poles 2 beschriebene Anordnung könnte bei einem Herzschrittmacher-Katheter mit mehr als zwei Polen entsprechend oft wiederholt sein. Sowohl bei koaxial aufgebauten Elektrodenwendeln als auch bei parallel zueinander in axialer Richtung und mit gleichem Außendurchmesser hergestellten mehrpoligen Elektrodenzuleitungen kann also eine sichere, elektrisch leitende Verbindung zwischen dem proximalen Pol und der Elektrode und der Zuleitungswendel über das Metallband 6 erfolgen.

Der Herzschrittmacher-Katheter 1 mit wenigstens zwei in axialer Richtung beabstandeten Polen und mit innerhalb von Isolierungen zu diesen Polen führenden Wendeln 3 und 4 oder dergleichen Zuleitungen hat zumindest einen ersten Pol 2, der durch einen Metallring oder eine Pol-Hülse 5 gebildet und mit der entsprechenden Zuleitung elektrisch leitend verbunden ist. Diese Verbindung ist durch wenigstens ein elektrisch leitendes Metallband 6 hergestellt, welches einerseits mit der zu der Pol-Hülse 5 gehörenden Zuleitung oder Wendel 6 und andererseits mit der Pol-Hülse 5 verbunden ist, so daß eine Löt- oder Schweißverbindung für eine sichere Kontaktgabe hoher Festigkeit möglich ist und durch die Flexibilität des Metallbandes auch dynamische Belastungen im Pol-Bereich ohne Auswirkung auf diese elektrische Verbindung bleiben.

**Patentansprüche**

1. Herzschrittmacher-Katheter (1) mit zumindest zwei voneinander in axialer Richtung beabstandeten Reizelektroden oder Polen sowie mit zumindest zwei innerhalb einer Isolierung (9) zu den Polen geführten, zu einer Wendel gewickelten und gegeneinander isolierten Zuleitungen, deren Windungen jeweils parallel und koaxial zueinander angeordnet sind, wobei ein erster Pol (2) durch einen leitenden Metallring oder eine Pol-Hülse (5) gebildet ist, der mit einer der Zuleitungen elektrisch leitend verbunden ist und gegenüber den Zuleitungen zu dem zweiten Pol isoliert ist, **dadurch gekennzeichnet**, daß wenigstens ein elektrisch leitendes Band oder Metallband einerseits in elektrischem Kontakt mit Windungen der zu der Pol-Hülse (5) gehörenden Zuleitung oder Wendel (3) vorgesehen und andererseits mit dieser Pol-Hülse (5) oder deren Innenseite verbunden ist.

2. Herzschrittmacher-Katheter nach Anspruch 1, dadurch gekennzeichnet, daß innerhalb der Pol-Hülse (5) eine Stütz- oder Haltehülse (7) angeordnet ist, mit der das innere Ende des Bandes oder Metallbandes (6) elektrisch leitend verbunden ist, und daß das Band (6) zwischen einem etwa in axialer Richtung an dieser Haltehülse (7) verlaufenden Abschnitt (6a) und einem zweiten, an der Pol-Hülse (5) oder deren Innenseite verlaufenden Abschnitt (6b) einen etwa Z- oder S-förmigen Übergang aufweist.

3. Herzschrittmacher-Katheter nach Anspruch 1 oder 2, dadurch gekennzeichnet, daß die Haltehülse (7) etwa gleich lang oder gegebenenfalls wenig kürzer oder länger als die Pol-Hülse (5) ist und als innere Armierung des Pol-Bereiches insbesondere beim Aufpressen der Pol-Hülse (5) dient.

4. Herzschrittmacher-Katheter nach einem der Ansprüche 1 bis 3, dadurch gekennzeichnet, daß für jeden der Pole eine eigene wendelförmige Zuleitung vorgesehen ist und beide Wendeln (3, 4) koaxial ineinander verlaufen, wobei eine Isolierung (8) zwischen ihnen angordnet ist, daß die auf größerem Durchmesser verlaufende Wendel (3) zu dem proximalen, als Pol-Hülse (5) gestalteten Pol (2) gehört und in ihrem Endbereich innerhalb des Poles (2) die Haltehülse (7) zwischen sich und der Isolierung (8) zu der inneren Wendel (4) aufnimmt und daß das Metallband (6) zwischen dieser äußeren Wendel (3) und der Haltehülse (7) an der Haltehülse (7) und insbesondere mehreren Windungen der Wendel (3) anliegt und zwischen zwei Windungen und durch die äußere Isolierung (9) der äußeren Wendel (3) an die Innenseite der Pol-Hülse (5) geführt ist.

5. Herzschrittmacher-Katheter nach einem der Ansprüche 1 bis 3, dadurch gekennzeichnet, daß die Windungen (3, 4) der beiden Zuleitungen denselben Außendurchmesser aufweisen und gegeneinander isoliert sind, daß im Bereich des proximalen Poles (2) die Haltehülse (7) innerhalb der Mehrfachwendel angeordnet ist, daß die zu dem proximalen Pol (2) führende Zuleitung (3) zumindest im Bereich der Haltehülse (7) abisoliert und mit dieser elektrisch leitend verbunden ist und daß das Metallband (6) mit der Haltehülse (7) außenseitig verbunden und zwischen gegebenenfalls isolierten Windungen radial nach außen an die Innenseite der äußeren Pol-Hülse (5) geführt ist.

6. Herzschrittmacher-Katheter nach Anspruch 5, dadurch gekennzeichnet, daß im Bereich der Haltehülse (7) zwischen dieser und den Windungen der Zuleitungen (3, 4) ein etwa über die Länge der Haltehülse (7) reichender Isolier-Überzug (10) vorgesehen ist, der im Bereich der Verbindung mit dem Ende (3a) der Zuleitung (3) zu der Haltehülse (7) durchbrochen ist, und daß das Metallband (6) zwischen diesem Isolier-Überzug (10) und der Außenseite der Haltehülse (7) angeordnet ist und durch den Isolier-Überzug (10) nach außen zu der Innenseite der Pol-Hülse (5) verläuft.

7. Herzschrittmacher-Katheter nach einem der Ansprüche 1 bis 3, dadurch gekennzeichnet, daß die innere Haltehülse (7) an der Außenseite der gemeinsamen Isolierung (9) der als Mehrfachwendel gewickelten Windungen der verschiedenen Zuleitungen angeordnet ist, daß die zu dem proximalen Pol (2) führenden Windungen (3) durch die gemeinsame Isolierung (9) hindurchgeführt und mit der Außenseite der Haltehülse (7) verbunden sind, mit der gleichzeitig das Metallband (6) in Kontakt ist, und daß das Metallband (6) durch eine äußere Isolierung (12) der Haltehülse (7) zu der Innenseite der Pol-Hülse (5) verläuft.

8. Herzschrittmacher-Katheter nach einem der Ansprüche 1 bis 7, dadurch gekennzeichnet, daß das Metallband (6) mit der Haltehülse (7) und/oder der Innenseite der Pol-Hülse (5) verschweißt oder verlötet ist.

9. Herzschrittmacher-Katheter nach einem der Ansprüche 1 bis 8, dadurch gekennzeichnet, daß die Löt- oder Schweißstelle (3) zum Verbinden des Metallbandes (6) mit der Pol-Hülse (5) nahe dem stirnseitigen Rand (14) der Pol-Hülse (5) angeordnet ist.

10. Herzschrittmacher-Katheter nach einem der Ansprüche 1 bis 9, dadurch gekennzeichnet, daß das Ende der zu dem proximalen Pol (2) führenden Zuleitung (3) oder Zuleitungen mit der Haltehülse (7) verschweißt ist.

11. Herzschrittmacher-Katheter mit mehr als zwei Polen nach einem der Ansprüche 1 bis 10, dadurch gekennzeichnet, daß eine weitere, insbesondere wenigstens aus einem Draht zu einer Wendel gewickelte Zuleitung vorgesehen ist, die zu einem dem proximalen Pol (2) benachbarten weiteren Pol führt und über ein weiteres Metallband (6) und vorzugsweise eine innere Stützhülse (7) mit einer äußeren Pol-Hülse (5) verbunden ist.

Fig. 1

Fig. 2

Fig. 3

Fig. 4

Fig. 5

Fig. 6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-4590950 (IWASZKIEWICZ)<br>* Spalte 4, Zeilen 29 - 30 *<br>* Spalte 4, Zeilen 40 - 43 *<br>* Spalte 5, Zeilen 27 - 47; Figur 2 * | 1 | A61N1/05 |
| A | | 2 | |
| | --- | | |
| A | DE-A-3640033 (SIEMENS)<br>* Spalte 2, Zeilen 45 - 61 * | 1 | |
| | --- | | |
| A | US-A-4387727 (SANDSTROM)<br>* Zusammenfassung; Figur 2 *<br>* Spalte 3, Zeilen 25 - 46 * | 1 | |
| | --- | | |
| A | US-A-4628943 (MILLER)<br>* Spalte 7, Zeilen 53 - 57 * | 1-5, 7, 11 | |
| | --- | | |
| A | FR-A-2491763 (MEDTRONIC)<br>* Ansprüche 11-13 * | 8, 10 | |
| | ----- | | |

| | RECHERCHIERTE SACHGEBIETE (Int. Cl.5 ) |
|---|---|
| | A61N |

Der vorliegende Recherchenbericht wurde für alle Patentansprüche erstellt

| Recherchenort | Abschlußdatum der Recherche | Prüfer |
|---|---|---|
| DEN HAAG | 10 SEPTEMBER 1991 | TACCOEN J-F.P.L. |

EPO FORM 1503 03.82 (P0403)